(19) Europäisches Patentamt
European Patent Office
Office européen des brevets

(11) Veröffentlichungsnummer : **0 426 176 B1**

# (12) EUROPÄISCHE PATENTSCHRIFT

(45) Veröffentlichungstag der Patentschrift :
**23.03.94 Patentblatt 94/12**

(51) Int. Cl.$^5$ : **C07H 15/26**

(21) Anmeldenummer : **90120948.6**

(22) Anmeldetag : **31.10.90**

(54) **5-(alpha-D-Glucopyranosyloxymethyl)-furan-2-carboxaldehyd und dessen Derivate und Folgeprodukte sowie Verfahren zur Herstellung der Verbindungen und deren Verwendung.**

(30) Priorität : **02.11.89 DE 3936522**

(43) Veröffentlichungstag der Anmeldung :
**08.05.91 Patentblatt 91/19**

(45) Bekanntmachung des Hinweises auf die Patenterteilung :
**23.03.94 Patentblatt 94/12**

(84) Benannte Vertragsstaaten :
**AT BE CH DE DK ES FR GB GR IT LI LU NL SE**

(56) Entgegenhaltungen :
**EP-A- 0 230 250**
**CHEMICAL ABSTRACTS, Band 79, Nr. 7, 20. August 1973, Seite 387, Zusammenfassung Nr. 42782t, Columbus, Ohio, US; G.O. ASPINALL et al.: "Base-catalyzed degradations of carbohydrates. V. Conversion of 3-deoxyhex-2-enopyranoses into 2-furoic esters", & CAN. J. CHEM. 1973, 51(9), 1359-62**
**CHEMICAL ABSTRACTS, Band 94, Nr. 19, 11. Mai 1981, Seite 633, Zusammenfassung Nr. 156646s, Columbus, Ohio, US; Y. NAKAMURA: "Preparation of 5-(hydroxymethyl)furfural by selective dehydration of D-fructose", & NOGUCHI KENKYUSHO JIHO 1980, (23), 25-38**
**CHEMICAL ABSTRACTS, Band 82, Nr. 15, 14. April 1975, seite 464, Zusammenfassung Nr. 98278n, Columbus, Ohio, US; R.A. SCHRAUFNAGEL et al.: "Levulinic acid from sucrose using acidic ion-exchange resins", & IND. ENG. CHEM., PROD. RES. DEV. 1975, 14(1), 40-4**
**BULLETIN DE LA SOCIETE CHIMIQUE DE FRANCE, no. 5, 1987, pages 855-860; T. EL HAJJ et al.: "Synthèse de l'hydroxyméthyl-5 furanne carboxaldéhyde-2 et de ses dérivés par traitement acide de sucres sur résines échangeuses d'ions"**

(56) Entgegenhaltungen :
**CHEMICAL ABSTRACTS, Band 109, Nr. 15, 10. Oktober 1988, Seite 543, Zusammenfassung Nr. 127407n, Columbus, Ohio, US; T. URASHIMA et al.: "The condensation of 5-(hydroxymethyl)-2-furfuraldehyde with some aldoses on heating", & FOOD CHEM. 1988, 29(1), 7-17 Food Chemistry 1988, vol. 29, p.7-17; Can J Chem. 1973, vol 51, p. 1359-62**

(73) Patentinhaber : **SÜDZUCKER AKTIENGESELLSCHAFT MANNHEIM/OCHSENFURT**
**Maximilianstrasse 10**
**D-68165 Mannheim (DE)**

(72) Erfinder : **Lichtenthaler, Frieder W., Prof.-Dr.**
**Am Willgraben 5**
**W-6109 Mühtal (DE)**
Erfinder : **Martin, Dierk, Cand. Ing.**
**Forsthausstrasse 47**
**W-6057 Dietzebach (DE)**
Erfinder : **Weber, Thomas A., Dipl.-Ing.**
**Schnorrenbach (Kreis Bergstrasse)**
**W-6943 Birkenau (DE)**
Erfinder : **Schiweck, Hubert M., Dr.**
**John-F.-Kennedy-Strasse 5**
**W-6520 Worms (DE)**

(74) Vertreter : **Gleiss, Alf-Olav, Dipl.-Ing.**
**Gleiss & Grosse Patentanwaltskanzlei**
**Maybachstrasse 6A**
**D-70469 Stuttgart (DE)**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 426 176 B1

Jouve, 18, rue Saint-Denis, 75001 PARIS

## Beschreibung

### 5-(α-D-Glucopyranosyloxymethyl)-furan-2-carboxaldehyd: Verfahren zur Herstellung aus Isomaltulose, Folgeprodukte und deren Verwendung

Die vorliegende Erfindung betrifft ein neues Verfahren zur Herstellung von 5-(α-D-Glucopyranosyloxymethyl)-furan-2-carboxaldehyd (im nachfolgenden auch als 5-Glucosyloxyrethyl-furfural oder GMF bezeichnet) und dessen Derivate und Folgeprodukte der allgemeinen Formel (I)

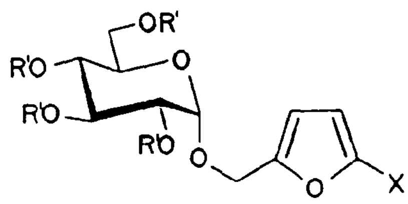

(I)

in der R' ein Wasserstoffatom, einen Acyl-Rest oder einen Alkyl-Rest darstellt und X-$CH_2OH$, -COOH, -C(H)=NOH, -CN, -$CH_2NHR$", oder CH=CR"R"' 1 bedeutet,
wobei R" ein Wasserstoffatom, ein Acyl-Rest, Aryl-Rest oder Alkyl-Rest mit bis zu 20 C-Atomen ist,
R"' ein Wasserstoffatom -$NO_2$, -CN, -COOAlkyl oder Acyl.

Die Acyl-Reste stammen von aliphatischen oder aromatischen Carbonsäuren; typische Vertreter sind die O-Acetyl- und O-Benzoyl-Derivate.

Typische Beispiele für Alkyl-Reste sind die Methyl-, Ethyl- und Octyl-Gruppierungen.

Die Verbindungen gemäß der obigen Formel (I) sind neu; sie finden u.a. als Zwischenprodukte für chemische Synthesen, beispielsweise zur Herstellung von oberflächenaktiven Verbindungen, technische Anwendung.

Das Ausgangsmaterial für die Herstellung der Verbindungen der oben angegebenen allgemeinen Formel bildet die Isomaltulose, d.i. die D-Glucopyranosyl-α(1-->6)-D-fructofuranose der unten angegebenen Formel (**1**), die bei Erhitzen in polaren, aprotischen Lösungsmitteln, wie z.B. Dimethylsulfoxid, in Gegenwart eines schwach oder stark sauren Ionenaustauschers in der $H^+$-Form oder anderer saurer Katalysatoren einer ausschließlich im Fructosyl-Teil erfolgenden dreifachen Wasserabspaltung unterliegt. Produkt dieser hochselektiven Dehydratisierung ist der 5-(α-D-Glucopyranosyloxymethyl)-furan-2-carboxaldehyd der Formel (**2**) in nachstehendem Formelschema.

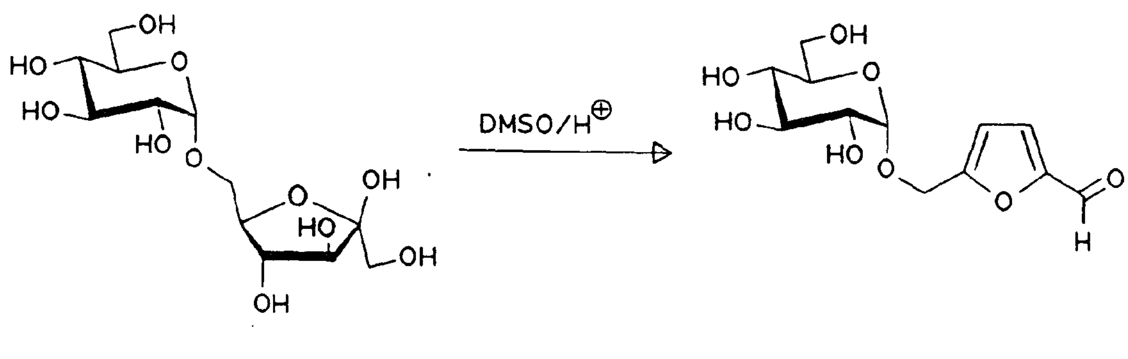


(**1**) (**2**)

Zur Überführung in das 5-Glucosyloxymethyl-furfural der Formel (**2**) wird die Isomaltulose wasserfrei (Formel **1**) oder als Monohydrat in einem stark polaren, aprotischen Lösungsmittel wie z.B. Dimethylsulfoxid, N,N-Dimethylformamid, Hexamethylphosphorsäure-triamid (HMPT), N-Methylpyrrolidon, oder einem Gemisch dieser Solventien, gelöst, und in Gegenwart eines sauren Ionenaustauschers wie z.B. Amberlite IR-120 oder Dowex 50 WX 4, jeweils in der $H^+$-Form, auf 70 bis 150°C für 0.1 - 24 h erhitzt. Bevorzugte Bedingungen sind

hierbei Erhitzen für 4 h bei 120°C in Gegenwart eines stark sauren Ionenaustauschers (Amberlite IR-120, $H^+$-Form, getrocknet) , oder 3 h bei 130°C in Anwesenheit von Dowex 50 WX 4($H^+$-Form, getrocknet); das so erhaltene Reaktionsgemisch enthält nach chromatographischer Analyse (DG bzw. HPLC) 65 - 70% GMF, 5 - 10% Glucose und HMF (entstanden durch glycosidische Spaltung des gebildeten GMF), 5 - 10% Isomaltulose-Dimere und 10 - 15% Edukt. Längere Reaktionszeiten verringern zwar die Menge nicht umgesetzter Isomaltulose, lassen jedoch den Gehalt an Glucose und HMF ansteigen (bis zu 20% nach 10 h), während der Anteil an Isomaltulose-Dimeren unverändert bleibt.

Alternativ kann Isomaltulose dadurch in 65 - 70% GMF enthaltende Gemische obiger Zusammensetzung übergeführt werden, daß sie wasserfrei oder mit einem Mol Kristallwasser, in Dimethylsulfoxid oder anderen stark polaren aprotischen Lösungsmitteln gelöst (10 - 100g / 100g Lösungsmittel) über eine mit den gleichen stark sauren Ionenaustauschern gefüllte Säule bei Temperaturen von 70 - 150°C perkoliert, mit Verweilzeiten zwischen 20 sec und min. Eine Erschöpfung des Katalysators konnte hierbei nicht festgestellt werden.

Zur Isolierung des gebildeten GMF der Formel (**2**) wird vom Katalysator abfiltriert, das Lösungsmittel i.Vak. abgezogen und der verbleibende Sirup durch Elution von einer Kieselgel- oder Ionenaustauscher-Säule aufgetrennt. Zunächst werden Dimere der Isomaltulose und Isomaltulose selbst eluiert, danach GMF, das in Form eines hochviskosen Sirups anfällt, in Ausbeuten, die durchweg über 65% liegen (vgl. Beispiel 1: 68% der Theorie). In kristalliner Form kann GMF als 2,4-Dinitrophenylhydrazon charakterisiert werden. Wird die zuletzt eluierte, aus unumgesetzter Isomaltulose und ihren Dimeren bestehende Fraktion i.Vak. zur Trockne eingedampft, und nochmals dem Batchbetrieb oder dem kontinuierlichen Prozeß im Durchflußreaktor unterworfen, lassen sich nach analoger Aufarbeitung weitere 15 - 20% GMF isolieren, was die Gesamtausbeute Isomaltulose --> GMF auf 80 - 85% der Theorie erhöht.

An sich ist bei Ketosen die dreifache Abspaltung von Wasser zur Bildung von furanoiden Produkten bekannt, wie der Übersicht von H.E. van Dam, A.P.G. Kieboom und H. van Bekkum in *Stärke* **38** (1986), S. 95 ff. und der dort zitierten Literatur zu entnehmen ist; Fructose z.B., läßt sich in guten Ausbeuten in 5-Hydroxymethyl-furfural (HMF) überführen, u.a. durch Erhitzen in aprotischen Lösungsmitteln in Gegenwart saurer Ionenaustauscher. Wie die Angaben von A. Gaset et al. in FR 2 551 754 A1 ausweisen, wird hierbei jedoch in der Praxis ein Verhältnis Fructose : Ionenaustauscher von 1 : mindestens 1 benötigt. Die Übertragung derartiger Bedingungen auf Isomaltulose würde in beträchtlichem Ausmaß zur Hydrolyse GMF -> HMF + Glucose führen. Dies dürfte auch der Grund sein, daß die selektive Dehydratisierung von Ketosen im Oligosaccharid-Verband zu den entsprechenden Analogen des GMF bislang unbekannt ist. Daß dies im Falle der Isomaltulose unter vergleichsweise milden Bedingungen selektiv durchgeführt werden kann, d.h. ohne extensive Spaltung der glycosidischen Bindung im Molekül, ist in hohem Maße überraschend, und unter anderem darauf zurückzuführen, daß bei der Umsetzung selbst mit einem Verhältnis Isomaltulose : Ionenaustauscher von 20 : 1 gearbeitet werden kann.

Zwar ist das GMF der Formel (**2**) schon 1988 in einer japanischen Arbeit erwähnt worden: T. Urashima, K. Suyama und S. Adachi berichten in *Food Chem.* **29** (1988), S. 7 - 17, über das Erhitzen von Hydroxymethylfurfural und D-Glucose in Dioxan für 6 h auf 150°C. Aus dem dabei entstehenden Gemisch von mindestens **4** chromatographisch nachweisbaren, neuen Substanzen wurde durch aufwendige Trennung (präparative Dünnschichtchromatopraphie) eine Reihe sirupöser Produkte isoliert, deren eines nach $^{13}C$-NMR-Daten das GMF der Formel (**2**) sein soll. Obwohl das Vorhandensein von GMF der Formel (**2**) in dem entstandenen Gemisch bzw. in einer der sirupösen Trennfraktionen nicht ausgeschlossen werden kann, ist der von den japanischen Autoren gegebene Strukturbeweis nicht schlüssig, da ihr GMF nicht kristallisiert.

Demgegenüber finden wir, daß das von uns aus Isomaltulose hergestellte GMF außerordentlich leicht aus Aceton oder Wasser kristallisiert und besonders schön ausgebildete Kristalle (Prismen) ergibt.

Durch Weiterverarbeitung des aus Isomaltulose der Formel (**1**) gewonnen GMF der Formel (**2**) oder seiner Derivate mittels Reduktion, Oxidation, Kondensationen des Aldol-Typs oder Oximierung wird der zugehörige Alkohol, die Carbonsäure, C-Extensionsprodukte oder das Oxim der Formeln (**3**), (**4**), (**9**), (**15**) und (**16**) erhalten.

Die O-Acylderivate, insbesondere die O-Acetyl- und O-Benzoylderivate des vorstehend angeführten Glucosyloxymethyl-furfurals und seiner Abkömmlinge können durch Behandeln der jeweiligen nicht O-acylierten Ausgangsverbindungen mit Acylierungsmitteln in wasserfreien organischen Lösungsmitteln erhalten werden.

Die Abläufe der genannten Umsetzungen und Weiterverarbeitungen sowie einige der dabei entstehenden Produkte sind aus nachstehendem Formelschema ersichtlich.

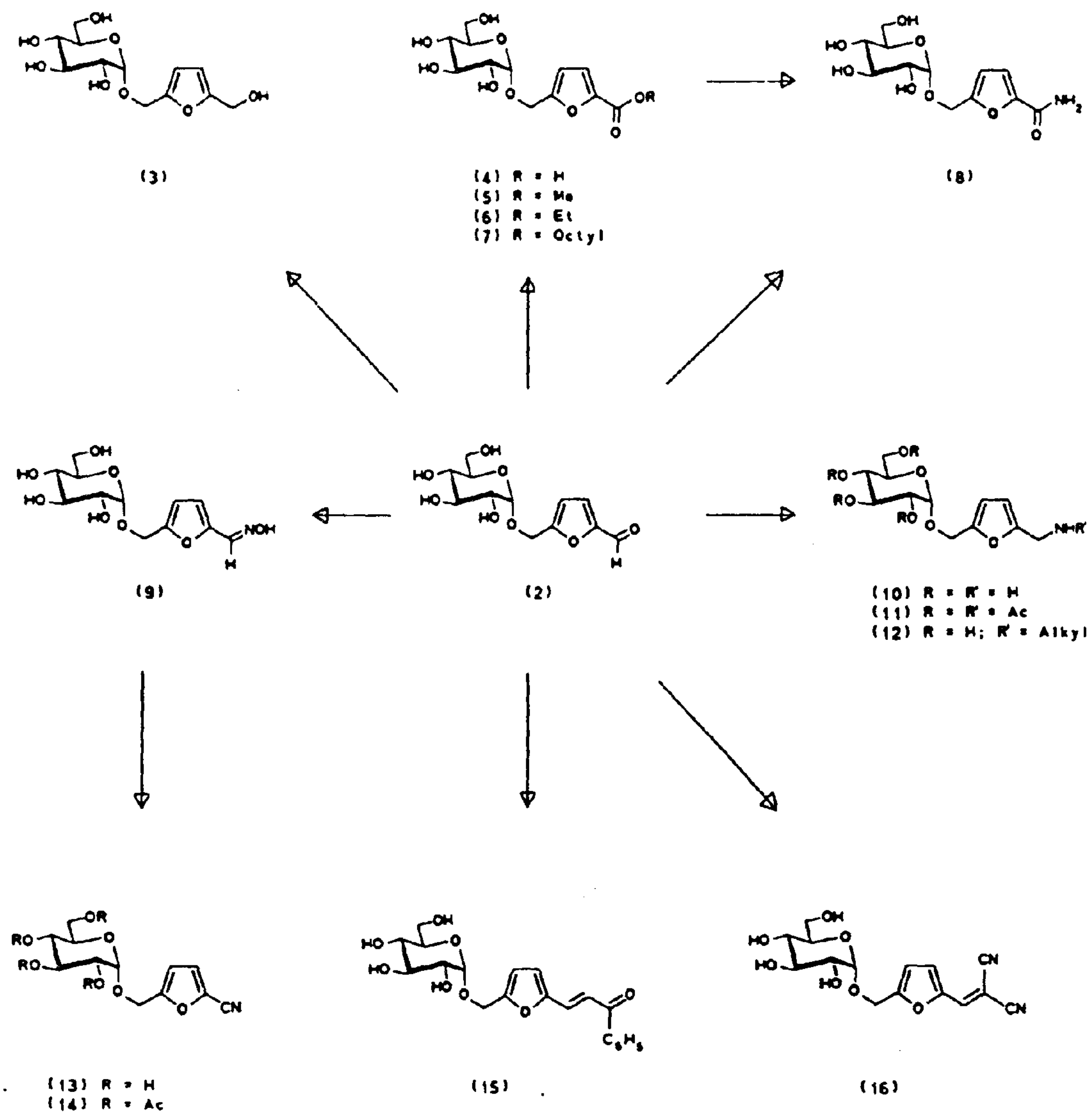

(3)

(4) R = H
(5) R = Me
(6) R = Et
(7) R = Octyl

(8)

(9)

(2)

(10) R = R' = H
(11) R = R' = Ac
(12) R = H; R' = Alkyl

(13) R = H
(14) R = Ac

(15)

(16)

Die Darstellung von Derivaten des Glucosyloxymethylfurfurals (GMF, Formel **2**) , z.B. des Tetraacetats ist unter den üblichen Bedingungen (Acetanhydrid/Pyridin) ohne weiteres durchführbar (vgl. Beispiel 2). Ebenso verläuft die Reduktion der Aldehyd-Funktion des GMF zum 2-Glucosyloxymethyl-5-hydroxymethylfuran der Formel (**3**) glatt mit einer ganzen Reihe unterschiedlicher Verfahren: Hydrid-Addition durch Behandeln mit Natriumborhydrid in Methanol oder Druckhydrierung mit Cu/Cr-Katalysator bei 140°C/100 bar oder gekreuzte Cannizzaro-Reaktion bei Umsetzung mit Formaldehyd in Natronlauge.

Die selektive Oxidation der Aldehyd-Gruppe des GMF zur Carbonsäure-Funktion erfolgt bei Umsatz mit Natriumchlorit in einer durch Kaliumdihydrogenphosphat gepufferten wäßrigen Lösung ohne jegliche Beeinträchtigung der vier Hydroxyl-Gruppen im Glycosyl-Teil. Das dabei entstehende freie Chlor wird mit Amidoschwefelsäure abgefangen. Die Reaktion verläuft rasch, ist nach vollständiger Zugabe des Oxidans bereits beendet, und liefert die 5-Glucosyloxymethylfuran-2-carbonsäure der Formel (**4**) in 80proz. Ausbeute.

Die GMF-Carbonsäure der Formel (**4**) läßt sich glatt verestern, wie durch die Darstellung des Methyl- (Formel **5**), Ehtyl- (Formel **6**) und Octyl-esters (Formel **7**) belegt wird (vgl. Beispiele 5 - 7). Desweiteren kann der Methylester durch Ammonolyse glatt in das schön kristalline GMF-Carbonamid der Formel (**8**) übergeführt werden.

Das GMF-Oxim der Formel (**9**) bildet sich durch Umsetzung von GMF mit Hydroxylammoniumchlorid/Natriumacetat in wäßrig-ethanolischer Lösung und fällt an in Form schön ausgebildeter Kristalle in 71proz. Ausbeute. Umsetzung des GMF-Oxims der Formel (**9**) mit Acetanhydrid führt zunächst zur Penta-O-acetyl-Verbindung, die bei Verschärfung der Bedingungen (Erwärmen) unter Abspaltung von Essigsäure glatt (78%) in das Nitril der Formel (**14**) übergeht. Dessen Hydrierung über Raney-Nickel in Essigester liefert das entsprechende Amin, das als N-Acetat (Formel **11**) in kristalliner Form charakterisiert wurde. Das freie Amin der Formel (**10**) kann direkt aus GMF durch reduktive Aminierung erhalten werden, indem man GMF in methanolischem Ammoniak mit Raney-Nickel einer Druckhydrierung (150 bar) unterwirft. Bei Einsatz primärer Amine (anstelle Ammoniak), wie z.B. Methylamin oder Dodecylamin, werden die entsprechenden N-Alkylamino-Verbindungen der Formel (**12**) erhalten in Ausbeuten, die generell über 80% liegen.

Die Aldehydgruppe des GMF gibt zwanglos Folgereaktionen des Typs der Aldol-Kondensation unter Bildung der entsprechenden Carbonyl-Olefinierungsprodukte; so entsteht bei der Umsetzung von GMF mit Acetophenon in Gegenwart von NaOH glatt das Benzoylvinyl-substituierte Derivat der Formel (**15**), oder bei Reaktion mit Malodinitril das Dicyanovinyl-Analogon der Formel (**16**).

Alle aus dem Formelschema ersichtlichen, erstmals dargestellten Verbindungen, das sind die Produkte der Formel (**3**) bis (**16**) wurden mikroanalytisch und chromatographisch einhellig als Reinsubstanzen charakterisiert; ihre Konstitution und Konfiguration wurde durch 300 MHz-$^1$H-NMR-Spektren sowie anhand $^{13}$C-NMR- und massenspektroskopischer Daten zweifelsfrei bewiesen.

Die Erfindung wird anhand der folgenden Beispiele näher erläutert.

**Beispiel 1**

**5-(α-D-Glucopyranosyloxymethyl)-furan-2-carboxaldehyd** (Formel **2**)

[5-(α-D-Glucopyranosyloxymethyl)-furfural, GMF]

**Methode A:** In einem 1.5 1 Rundkolben wird eine Lösung von 90g (0,25 mol) Isomaltulose-/Monohydrat (Formel **1**) in 900ml Dimethylsulfoxid auf 120°C erhitzt, sodann mit 10g eines stark sauren Ionenaustauschers (Amberlite IR 120, H+-Form, i.Vak. getrocknet über $P_2O_5$ für 24 h) versetzt und das Gemisch unter Rühren 4 h bei 120°C gehalten; nach DC [Laufmittel: Acetonitril/Wasser (4:1); $R_f$-Werte: 0.10 (Dimere der Isomaltulose), 0.15 (Isomaltulose), 0.26 (Glucose), 0.48 (GMF), 0.61 (HMF)] sowie HPLC, besteht das Gemisch aus 65 - 70% GMF, ca. 10% Isomaltulose-Dimeren, 5 - 10% HMF + Glucose und ca. 10% Edukt. Zur Aufarbeitung wird vom Ionenaustauscher abfiltriert und das Lösungsmittel i.Vak. bei 80°C Badtemperatur abgezogen. Der verbleibende braune Sirup wird in Wasser (250ml) gelöst, auf eine auf 60°C vorgeheizte Lewatit TSW 40-Säule ($Ca^{2+}$-Form, 8 x 200cm) aufgegeben, und mit Wasser (10 l/h) eluiert. Die nach einem Vorlauf von ca. 3 l Wasser erscheinende erste Fraktion (2 l) enthielt die Dimere der Isomaltulose und nicht umgesetzte Isomaltulose (Weiterverarbeitung dieser Fraktion vide infra); danach wird Glucose eluiert, gefolgt von GMF (ca. 6 l). Einengen der GMF enthaltenden Eluate liefert 49g (68 % der Theorie) eines chromatographisch und $^1$H-NMR-spektroskopisch einheitlichen viskosen Sirups mit $[\alpha]_D^{20}$ = + 125° (c= 1.3, Methanol) der für Folgereaktionen direkt verwendet werden kann.

Reinigung des Rohproduktes durch Elution von einer Kieselgelsäule (3 x 30cm für 5g Sirup) mit Aceton und Einengen des Eluats liefert zunächst gelbliche Kristalle vom Schmp. 88 - 89°C; Nach zweifacher Umkristallisation wird das GMF in Form schön ausgebildeter farbloser Prismen vom Schmp. 96°C und $[\alpha]_D^{20}$ = + 131° (c= 1, Methanol) erhalten.

$^1$H-NMR (300 MHz, DMSO-$d_6$): δ = 3.12 (m, 1 H, 4'-H), 3.25 (m, 1H, 2'-H), 3.38 - 3.64 (m, 4 H, 3'-H, 5'-H, 6'-$H_2$) 4.54 (t, 1 H, 6'-OH), 4.56 (d, 1 H, 7-$H_a$), 4.69 (d, 1 H, 7-$H_b$), 4.80 (d, 1 H, 1'-H), 4.84 (d, 1 H, OH), 4.87 (d 1 H, OH), 4.95 (d, 1 H, OH), 6.77 (d, 1 H, 4 H), 7.53 (d, 1 H, 3-H), 9.59 (s, 1 H, CHO); $J_{3,4}$ = 3.3, $J_{7_a,7_b}$ = 13.5, $J_{1',2'}$ = 3.3, $J_{6',6'-OH}$ = 6.2 Hz.

$^{13}$C-NMR (75.5 MHz, DMSO-$d_6$): δ = 60.5 (C-7), 60.8 (C-6'), 70.1 (C-4'), 71.8 (C-2'), 73.1 (C-3', C-5'), 98.4 (C-1'), 112.0 (C-4), 124.2 (C-3), 152.2 (C-5), 158.0 (C-2), 178.3 (C-6).

MS (FD): m/z = 288 ($M^{\oplus}$), 289 ($M^{\oplus}$ +1).

| | | |
|---|---|---|
| $C_{12}H_{16}O_8$ (288.25) | Ber. | C 50.00 H 5.60 |
| | Gef. | C 49.81 H 5.63 |

Wird die zuerst, aus Isomaltulose und ihren Dimeren bestehende Fraktion (s. oben) zur Trockne eingedampft und der Rückstand, in DMSO-Lösung, obigen Reaktionsbedingungen unterworfen, werden nach chromatographischer Auftrennung weitere 11g (15% der Theorie) GMF erhalten (Gesamtausbeute 80%). Präparativ praktikabler ist allerdings die Zumischung dieser, Isomaltulose und ihre Dimere enthaltenden DMSO-Lösung zu dem dem Durchflußreaktor der Abbild. 1 zugeführten Material.

**Methode B**: Über eine in einer Reaktionskammer befindlichen, mit stark saurem Ionenaustauscher des Typs Dowex 50 XW 4 ($H^+$-Form) gefüllten Säule (1.6 x 25cm, Reaktor der Abbild. 1) wird bei 130°C mittels einer Pumpe eine Lösung von 250g Isomaltulose-Monohydrat in 1 1 DMSO geleitet, wobei die Durchflußgeschwindigkeit so gewählt wird, daß die Verweilzeit der Lösung am Ionenaustauscher 4 min nicht übersteigt. Die Auftrennung des beim Verlassen der Reaktionskammer gekühlten Reaktionsgemischs, das dem obigen Batch-Verfahren erhaltenen Gemisch weitgehend analog zusammengesetzt ist, erfolgt wie bei Methode A beschrieben, und liefert 138g (69% der Theorie) GMF der Formel (**2**).

GMF-Dinitrophenylhydrazon: Eine Lösung von 5.0 g (25 mmol) 2,4-Dinitrophenylhydrazin in 25 ml konz. Schwefelsäure wird vorsichtig mit 60 ml Wasser und 200 ml Ethanol verdünnt, und zu der noch heißen Lösung 2.9 g (10 mmol) GMF, in 25 ml Ethanol gelöst, zugegeben. Nach 24 h wird der tiefrote Niederschlag abfiltriert, mit Wasser gewaschen und aus Ethylacetat umkristallisiert: 2.5 g (54 %) oranger Kristalle; Schmp. 186°C; $[\alpha]_D^{20}$ = + 80.8° (c= 1, Dimethylsulfoxid).

$^1$H-NMR (300 MHz, DMSO-$d_6$): $\delta$ = 3.15 (dd, 1 H, 4'-H), 3.25 (dd, 1 H, 2'-H] , 3.4 - 3.7 (m, 4 H, 3'-H, 5'-H, 6 '$H_2$ ] , 4.7 (m, 4 H, 2'-OH, 3'-OH, 4'-OH, 6'-OH), 4.66 (d, 1 H, 7-$H_a$), 4.78 (d, 1 H, 7-$H_b$), 4.86 (d, 1 H, 1'-H), 6.78 (d, 1 H, 4-H), 7.11 (d, 1 H, 3-H), 7.61 (s, 1 H, 6-H), 7.96 (d, 1 H, 6"-H) , 8.37 (dd, 1 H, 5"-H), 8.83 (d, 1 H, 3"-H) , 12.72 (s, 1 H, N-H) ; $J_{3,4}$ = 3,5, $J_{7a,7b}$ = 13.6, $J_{1',2'}$ = 3.6, $J_{2',3'}$ = 9,7, $J_{3',4'}$ = 9.0, $J_{4',5'}$ = 9.2 $J_{3'',5''}$ = 2.5, $J_{5'',6''}$ = 9.6 Hz.

$^{13}$C-NMR (75,5 MHz, DMSO-$d_6$) : $\delta$ = 60.5 (C-6') , 60.9 (C-7) , 69.8 (C-4') , 71.6 (C-2') 72.9, 73.0 (2 C, C-5' , C-3') 98.7 (C-1'), 111.5 (C-4) , 116.0 (C-6") , 118.7 (C-3) , 122.7 (C-3") 129.6, 137.5, 144.4 (3 C, C-1", C-2", C-4"), 130.0 (C-5") , 131.5 (C-6) 147.0 (C-5), 155.2 (C-2).

$C_{18}H_{20}N_4O_{11}$ (468.4) Ber. C 46.16 H 4.30 N 11.96
Gef. C 46.23 H 4.29 N 11.84

**Methode C:** Eine doppelwandige Stahlsäule (2,5 x 80 cm) wird mit ca. 400 ml eines in DMSO vorgequollenen Katalysatorharzes z.B. des Typs K 2413 (Bayer) in der $H^+$-Form gefüllt und mit Siebplatten verschlossen.

Die Säule wird durch den Mantel mittels Umwälzthermostat und Temperierflüssigkeit auf 90°C aufgeheizt.

Über das Katalysatorharz wird mittels einer Pumpe eine Lösung von 9 kg Isomaltulose-Monohydrat in 9 kg DMSO geleitet, wobei die Durchflußgeschwindigkeit so gewählt wird, daß die Verweilzeit der Lösung am Katalysator ca. 30 min. beträgt. Der Gehalt an GMF im Eluat beträgt 50-60 % der Trockensubstanz.

Das Reaktionsgemisch wird im Hochvakuum bei einer Badtemperatur von 80-90°C weitgehend vom Lösungsmittel befreit. Der verbleibende Rückstand wird mit dem gleichen Volumen Wasser verdünnt und über eine Lewatit TSW 40-Säule ($Ca^{++}$-Form, Abmessungen: 0,25 x 12m) bei 65°C chromatographisch aufgetrennt.

Die GMF enthaltenden Fraktionen werden eingedampft bis zu einem Trockensubstanzgehalt von 80-85 %.

Durch langsames Abkühlen der wässrigen Lösung von 70°C mit einer Kühlrate von 1 $K \cdot h^{-1}$ erhält man GMF-Kristalle mit einer Reinheit von ca. 97 % (HPLC).

Die Ausbeute beträgt 2.1 kg (29 %, bezogen auf eingesetztes Isomaltulose-Monohydrat).

Weiteres Produkt kann aus der Mutterlauge gewonnen werden.

Durch Umkristallisation unter gleichen Bedingungen erhält man GMF in einer Reinheit von 100 (HPLC).

Schmelzp. 88°C; $[\alpha]_D^{20}$ = + 98° (c=1, $H_2O$), + 131° (c=1, Methanol).

**Beipiel 2**

**5-(2',3',4',6'-Tetra-O-acetyl-α-D-glucopyranosyloxymethyl)-furan-2-carboxaldehyd**

[GMF-Tetraacetat]

Eine Lösung von 60 mg (0.3 mmol) Glucosyloxymethyl-furfural (Formel **2**, Produkt von Beispiel 1) in 25 ml Pyridin wird bei 0°C mit 0.3 ml Acetanhydrid versetzt. Nach Erwärmen des Ansatzes auf Raumtemperatur und weiterem Rühren über 2 h wird mit Eiswasser hydrolysiert. Aufnehmen der Reaktionslösung in Chloroform, neutral Waschen mit 2 N Schwefelsäure, gesättigter Natriumhydrogencarbonat-Lösung und Wasser, Trocknen über Magnesiumsulfat und Abziehen des Lösungsmittels ergeben 98 mg (84 %) GMF-Tetraacetat als Sirup mit $[\alpha]_D^{20}$ = + 132,8° (c=2,7, Chloroform).

$^1$H-NMR (300 MHz,$CDCl_3$): $\delta$ = 1.94, 1.96, 1.97, 2.02 (4 s, je 3 H, 4 Ac-$CH_3$), 3.99 (m, 2 H, 5'-H, 6'-$H_a$), 4.19 (dd, 1 H, 6'-$H_b$), 4.57 (d, 1 H, 7-$H_a$), 4.66 (d, 1 H, 7-$H_b$), 4.79 (dd, 1 H, 2'-H), 5.01 (dd, 1 H, 4'-H), 5.13 (d. 1 H, 1'-H), 5.41 (dd, 1 H, 3'-H), 6.50 (d, 1 H, 4-H), 7.16 (d, 1 H, 3-H], 9.56 (s, 1 H, CHO); $J_{3,4}$ = 3.5, $J_{7_a,7_b}$ = 13.5, $J_{1',2'}$ = 3.8, $J_{2',3'}$ = 10.3, $J_{3',4'}$ = 9.7, $J_{4',5'}$ = 9.6, $J_{5',6'}$ = 4.8, $J_{6'_a,6'_b}$ = 12.9 Hz.

$^{13}$C-NMR (75.5 MHz, $CDCl_3$): $\delta$ = 20.5, 20.6, 20.7, (4 C, Ac-$CH_3$), 61.6 (C-6'), 62.0 (C-7), 67.7 (C-5'), 68.3

(C-4'), 69.8 (C-3'), 70.6 (C-2'), 95.4 (C-1'), 112.1 (C-4), 121.9 (C-3), 152.9, 156.2 (2 C, C-5, C-2), 169.5, 170.0, 170.5 (4 C, 4 Ac-C=0), 177.6 (C-6).

MS (FD): m/z = 456 ($M^{\oplus}$), 457 ($M^{\oplus}$ +1).

**Beispiel 3**

**2-(α-D-Glucopyranosyloxymethyl)-5-hydroxymethyl-furan** (Formel **3**)

520 mg (1.8 mmol) Glucosyloxymethyl-furfural (Formel **2**, Produkt von Beispiel 1) werden in 30 ml Methanol gelöst und mit 138 mg (3.6 mmol) Natriumborhydrid versetzt. Nach 2 h Rühren bei Raumtemperatur zeigt das Dünnschichtchromatogramm kein Edukt mehr an. Zur Aufarbeitung wird das Lösungsmittel i. Vak. abgezogen und der verbleibende Sirup durch Elution mit Chloroform/Methanol (3:1) gereinigt. Nach Eindampfen der Fraktionen verbleiben 443 mg (85 %) Glucosyloxymethyl-hydroxymethylfuran der Formel (**3**) als farbloser Sirup mit $[\alpha]_D^{20}$ = +107° (c = 1, Methanol).

$^1$H-NMR (300 MHz, $CDCl_3$): δ = 3.13 (dd, 1 H, 4'-H), 3.27 (dd, 1 H, 2'-H), 3.41 - 3.70 (m, 4 H, 3'-H, 5'-H, 6'-$H_2$), 4.41 (d, 1 H, 7-$H_a$), 4.41 (s, 2 H, 6-$H_2$), 4.59 (d, 1 H, 7-$H_b$), 4.79 (d, 1 H, 1'-H), 5.2 (m, 5 H, 6-OH, 2'-OH, 3'-OH, 4'-OH, 6'-OH), 6.28 (d, 1 H, 4-H), 6.40 (d, 1 H, 3-H); $J_{3,4}$ = 2.8, $J_{7_a,7_b}$ = 12.8, $J_{1',2'}$ = 3.3, $J_{2',3'}$ = 9.6, $J_{3',4'}$ = 9.0, $J_{4',5'}$, = 9.0 Hz.

$^{13}$C-NMR (75.5 MHz, $CDCl_3$): δ = 56.1, 60.6, 61.3 (3 C, C-6, C-7, C-6'), 70.7, 72.2, 73.2, 73.6 (4 C, C-2', C-3', C-4', C-5'), 99.1 (C-1'), 107.9 (C-4), 110.5 (C-3), 150.9 (C-2), 156.0 (6-5).

MS (FD): m/z = 290 ($M^{\oplus}$).

**Beispiel 4**

**5-(α-D-Glucopyranosyloxymethyl)-furan-2-carbonsäure** (Formel **4**)

In 50 ml Wasser werden 3.16 g (11 mmol) GMF (Formel **2**, Produkt von Beispiel 1) und 2.13 g (22 mmol) Amidoschwefelsäure gelöst. Dazu tropft man innerhalb von 20 min 20 ml einer wäßrigen Lösung aus 1.0 g (11 mmol) Natriumchlorit und 1.1 g (8.1 mmol) Kaliumdihydrogenphosphat. Nach Zugabe der Oxidationslösung zeigt das Chromatogramm vollständigen Umsatz. Das Lösungsmittel wird i.Vak. entfernt und der verbleibende Sirup in einem Gemisch aus 40 ml Methanol und 40 ml Ethanol aufgenommen. Die ausfallenden anorganischen Salze werden abfiltriert und das Lösungsmittel erneut entfernt. Filtration des Rückstandes über eine Kieselgelsäule mit Methanol/Chloroform (2:1), und Eindampfen des Eluats liefert 2.9 g (89 %) Carbonsäure der Formel (**4**) als bislang nicht kristallisierender Feststoff.

Alternativ werden einer Lösung von 0.6 g (3.5 mmol) Silbernitrat in 10 ml Wasser unter Rühren 1.6 ml (4.0 mmol) 10proz. NaOH zugetropft. Das ausgefallene Silberoxid wird abfiltriert, mit 100 ml Wasser nitratfrei gewaschen und noch feucht in einen Kolben überführt, in dem es in 15 ml Wasser, das 0.7 ml (1.7 mmol) 10proz. NaOH enthält, suspendiert wird. Zu dieser Suspension gibt man unter heftigem Rühren 0.5 g (1.7 mmol) GMF, wobei ein Farbumschlag von braun nach schwarz und eine Temperaturerhöhung auf 30°C beobachtbar ist. Nach 15 min (DC-Kontrolle mit Laufmittel Acetonitril/Wasser (4:1), $R_f$ (Produkt) = 0.22) filtriert man vom Silber ab, neutralisiert das Filtrat mit saurem Ionenaustauscher (Amberlite IR-120, H -Form) und dampft i.Vak. ein. Der resultierende farblose Sirup wird an einer Kieselgelsäule (3 x 20 cm) durch Elution mit Chloroform/Methanol (2:3) gereinigt. Es fallen 460 mg (89%) chromatographisch sowie $^1$H-NMR-spektroskopisch einheitlicher, amorpher Feststoff mit $[\alpha]_D^{20}$ = +104° (c = 0.7, Methanol) an.

$^1$H-NMR (300 MHz, DMSO-$d_6$): δ = 3.06 (dd, 1 H, 4'-H), 3.19 (dd, 1 H, 2'-H), 3.41 (m, 4 H, 3'-H, 5'-H, 6'-$H_2$), 3.63 (d, 1 H, OH), 4.18 (m, 1 H, OH), 4.38 (d, 1 H, 7-$H_a$), 4.53 (d, 1H, 7-$H_b$), 4.74 (d, 1 H, 1'-H), 4.99 (m, 1 H, OH), 5.16 (m, 1 H, OH), 6.38 (d, 1 H, 4-H), 6.65 (d, 1 H, 3-H); $J_{3,4}$ = 3.5 $J_{7_a,7_b}$ = 12.8, $J_{1',2'}$ = 3.6, $J_{2',3'}$ = 9.4, $J_{3',4'}$ = 9.2 Hz.

$^{13}$C-NMR (75.5 MHz, DMSO-$d_6$): δ = 60.6 (C-7), 61.0 (C-6'), 70.4 (C-4'), 72.0 (C-2'), 73.2 (C-3', C5'), 98.2 (C-1'), 111.4 (C-4), 116.7 (C-3), 145.5 (C-5), 154.2 (C-2), 161.0 (C-6).

MS (FD, 10-15 mA): m/z = 305 ($M^{\oplus}$ + 1), 327 ($M^{\oplus}$ + Na).

| | | |
|---|---|---|
| $C_{12}H_{16}O_9$ (304.25) | Ber. C 47.37 H 5.30 |
| | Gef. C 47.30 H 5.27 |

**Beispiel 5**

**5(α-D-Glucopyranosyloxymethyl)-furan-2-carbonsäure-methylester** (Formel **5**)

Zur Darstellung von Diazomethan wird 1.0 g (10 mmol) N-Methyl-N-nitrosoharnstoff in 20 ml Diethylether, der mit 7 ml 40proz. KOH unterschichtet ist, unter Kühlung portionsweise so zugegeben, daß die Reaktionstemperatur +5°C nicht übersteigt. 30 min nach der letzten Zugabe wird die gelbe organische Phase abgetrennt und 3 h über wenig festem KOH stehen gelassen. Von der so bereiteten etherischen Diazomethan-Lösung wird unter Rühren bei Raumtemperatur über einen Zeitraum von 15 min soviel zu einer Lösung von 500 mg (1.6 mmol) GMF-Carbonsäure (Formel **4**, Produkt von Beispiel 4) in 18 ml Methanol und 2 ml Wasser gegeben, daß die gelbe Farbe gerade bestehen bleibt und keine Gasentwicklung mehr feststellbar ist. Nach weiteren 15 min Rühren ist die Umsetzung vervollständigt (DC-Kontrolle mit Laufmittel Chloroform/Methanol (2:1), $R_f$ (Edukt) = 0 , $R_f$ (Produkt) = 0.41), woraufhin man das Lösungsmittel i. Vak. abzieht und das Rohprodukt durch Elution von einer Kieselgelsäule (3 x 20 cm) mit Chloroform/Methanol (2:1) reinigt. Einengen der das Produkt enthaltenden Fraktionen ergibt 440 mg (84 %) farblosen Sirup mit $[\alpha]_D^{20}$ = +113° (c = 0.8, Methanol), der nach Ankratzen allmählich kristallisiert; Schmp. 72°C.

$^1$H-NMR (300 MHz, $D_2O$) δ = 3.44 (dd, 1 H, 4'-H), 3.57 (dd, 1 H, 2'-H), 3.66 (ddd, 1 H, 5'-H). 3.71 (dd, 1 H, 3'-H), 3.75 (d, 2 H, 6'-$H_2$) 3.91 (s, 3 H, $OCH_3$), 4.73 (s, 2 H, 7-$H_2$), 5.05 (d. 1 H, 1'-H), 6.67 (d, 1 H, 4-H), 7.29 (d, 1 H, 3-H); $J_{3,4}$ = 3.5, $J_{1',2'}$ = 3.7, $J_{2',3'}$ = 9.8, $J_{3',4'}$ = 9.3, $J_{4',5'}$ = 10.0, $J_{5',6'}$ = 3.6 Hz.

MS (FD, 0 - 15 mA): m/z = 318 ($M^{\oplus}$).

$C_{13}H_{18}O_9$ (318.28) Ber. C 49.06 H 5.70
Gef. C 48.72 H 5.65

**Beispiel 6**

**5-(α-D-Glucopyranosyloxymethyl)-furan-2-carbonsäureethylester** (Formel **6**)

Zu einer Lösung von 300 mg (1 mmol) GMF-Carbonsäure der Formel (**4**), 0.6 g (13.0 mmol) Ethanol und 20 mg *p*-Toluolsulfonsäure in 3 ml Pyridin werden 200 mg (1.0 mmol) Dicyclohexylcarbodiimid zugegeben, und 24 h bei Raumtemperatur gerührt. Nach Filtration wird i .Vak. vollständig eingeengt, mehrmals mit Toluol nachgedampft, und der anfallende Sirup wird über eine Kieselgelsäule (3 x 20 cm) durch Elution mit Chloroform/Methanol (7:2) gereinigt. Eindampfen der Fraktionen mit $R_f$ = 0.31 ergibt 190 mg (60 %) Ethylester der Formel (**6**) als farblosen Sirup, mit $[\alpha]_D^{20}$ = + 108° (c = 0.7, Methanol).

$^1$H-NMR (300 MHz, $D_2O$): δ = 1.37 (t, 3H, $O\underline{C}H_2CH_3$), 3.44 (dd, 1 H, 4'-H), 3.56 (dd, 1 H, 2'-H), 3.65 (ddd, 1 H, 5'-H), 3.70 (dd. 1 H, 3'-H), 3.75 (d, 2 H, 6'-$H_2$), 4.38 (q, 2H, $OCH_2CH_3$), $\underline{4}$.73 (s, 2 H, 7-$H_2$), 5.04 (d, 1 H, 1'-H), 6.67 (d, 1 H, 4-H), 7.30 (d, 1 H, 3-H);$J_{3,4}$ = 3.5, $J_{8,9}$ = 7.1, $J_{1',2'}$ = 3.8, $J_{2',3'}$ = 9.8, $J_{3',4'}$ = 9.3, $J_{4',5'}$ = 10.2, $J_{5',6'}$ = 3.6 Hz.

MS (FD, 0 - 12 mA): m/z = 332 ($M^{\oplus}$).

$C_{14}H_{20}O_9$ (332.31) Ber. C 50.60 H 6.07
Gef. C 49.91 H 6.31

**Beispiel 7**

**5-(α-D-Glucopyranosyloxymethyl)-furan-2-carbonsäureoctylester** (Formel 7)

Wird Ethanol in der vorhergehenden Vorschrift durch die equivalente Menge 1-Octanol ersetzt, so erhält man den entsprechenden Octylester der Formel (**7**) nach chromatographischer Reinigung (Elution mit Chloroform/Methanol (7:2)) in Form eines weißen amorphen Pulvers; $[\alpha]_D^{20}$ = + 86° (c = 0.7, Methanol).

$^1$H-NMR (300 MHz, DMSO-$d_6$): δ = 0.86 (t, 3 H, $CH_3$), 1.03 - 1.77 (m, 12 H, $[CH_2]_6$), 3.09 (m, 1 H, 2'-H), 3.23 (m, 1 H, 4'-H), 3.34 - 3.64 (m, 4 H, 3'-H, 5'-H, 6'-$H_2$), 4.23 (t, 2 H, $OCH_3$), 4.48 (m, 1 H, OH), 4.52 (d. 1 H, 7 $H_a$), 4.64 (d, 1 H, 7-$H_b$), 4.75 - 4.82 (m, 3 H, 1'-H, 2 OH), 4.90 (d, 1 H, OH), 6.56 (d, 1 H, 4-H), 6.87 (d, 1 H, 1'-H); $J_{3,4}$ = 3.4, $J_{7_a,7_b}$ = 13.4, $J_{8,9}$ = 6.7, $J_{14,15}$ = 6.7, $J_{1',2'}$ = 3.6 Hz.

MS (FD, 0 - 20 mA): m/z = 416 ($M^{\oplus}$), 417 ($M^{\oplus}$ +1).

$C_{20}H_{32}O_9$ (416.47) Ber. C 57.68 H 7.74
Gef. C 56.75 H 7.91

**Beispiel 8**

**5[α-D-Glucopyranosyloxymethyl)-furan-2-carbonsäureamid** (Formel **8**)

In 5 ml einer methanolischen Ammoniaklösung werden 300 mg (1.1 mmol) Methylester der Formel (**7**) gelöst. Nach 24stdg. Rühren bei Raumtemperatur ist die Aminolyse vollständig (DC-Kontrolle in Acetonitril/Wasser (4:1). Lösungsmittel und überschüssiges Ammoniak werden i. Vak. entfernt und der verbleibende weiße Feststoff kristallisiert beim Digerieren mit Methanol/Wasser: 270 mg (86 %) eines unter dem Mikroskop opaleszierenden Kristallpulvers vom Schmp. 218°C mit $[\alpha]_D^{20}$ = + 97° (c = 0.8, Methanol).

$^1$H-NMR (300 MHz, DMSO-$d_6$): 3.07 (m, 1 H, 4'-H), 3.23 (m, 1 H, 2'-H), 3.35-3.61 (m, 4 H, 3'-H, 5'-H, 6'-$H_2$), 4.47 (d, 1 H, 7-$H_a$), 4.53 (t, 1 H, 6'-OH), 4.60 (d, 1 H, 7-$H_b$), 4.76 (m, 3 H, 1'-H, 2 OH), 4.89 (d, 1 H, OH), 6.57 (d, 1 H, 4-H), 7.07 (d, 1 H, 3-H), 7.37 (s, 1 H, NH), 7.74 (s, 1 H, NH); $J_{3,4}$ = 3.3, $J_{7_a,7_b}$ = 13.2, $J_{6',6'\text{-}OH}$ = 5.7 Hz.

MS (FD, 0 - 20 mA): m/z = 303 ($M^{\oplus}$), 304 ($M^{\oplus}$ +1).

$C_{12}H_{17}NO_8$ (303.27) Ber. C 47.53 H 5.65 N 4.62
Gef. C 47.57 H 5.60 N 4.51

**Beispiel 9**

**5-(α-D-Glucopyranosyloxymethyl)-furan-2-carboxaldehyd-oxim** (Formel **9**) [GMF-Oxim]

Eine Lösung von 3.1 g (10.7 mmol) Glucosyloxymethyl-furfural (Formel **2**, Produkt. von Beispiel 1) in 50 ml Ethanol wird mit einer Lösung von 2.2 g (31.6 mmol) Hydroxylammoniumhydrochlorid und 2.1 g (25 mmol) Natriumacetat in 10 ml Wasser versetzt und 10 min bei Raumtemperatur gerührt. Danach zeigt das Dünnschichtchromatogramm (Laufmittel: Wasser/n-Propanol, 1:7) kein Edukt mehr an. Zur Aufarbeitung wird das Lösungsmittel i.Vak. entfernt und der zurückbleibende Sirup in 2-Propanol aufgenommen. Die dabei ausfallenden Salze werden abgenutscht. Ein erneutes Abziehen des Lösungsmittels ergibt einen leicht gefärbten Sirup, der zwecks weiterer Reinigung auf Kieselgel gegeben und mit Chloroform/Methanol (7:2) eluiert wird. Eindampfen der das GMF-Oxim (Formel **9**) enthaltenden Eluate, und Stehen des gelblichen Sirups liefert 2.3 g (71 %) farbloser Kristalle vom Schmp. 138 - 139°C; $[\alpha]_D^{20}$ = +118° (c = 1.5, Methanol).

$^1$H-NMR (300 MHz, $D_2O$): δ = 3.4 - 3.8 (m, 5 H, 2'-H, 3'-H, 4'-H, 6'-$H_2$), 3.57 (ddd, 1 H, 5'-H), 4.7 - 4.9 (m, 2 H, 7-$H_2$), 5.04 (d, 1 H, 1'-H), 6.66 (d, 1 H, 4-H), 7.27 (d, 1 H, 3-H), 8.06 (s, 1 H, 6-H); $J_{3,4}$ = 3.4, $J_{1',2'}$ = 3.6, $J_{4',5'}$ = 9.8, $J_{5',6'_a}$ = 1.3, $J_{5',6'_b}$ = 3.5 Hz.

$^{13}$C-NMR (75.5 MHz, $D_2O$): δ = 63.1, 64.3 (2 C, C-7, C-6'), 72.2 (C-4'), 74.0 (C-5'), 74.8 (C-2'), 75.8 (C-3'), 100.7 (C-1'), 115.3 (C-4), 121.8 (C-3), 140.4 (C-6), 147.9 (C-5), 155.2 (C-2).

MS (FD): m/z = 303 ($M^{\oplus}$), 304 ($M^{\oplus}$ +1).

$C_{12}H_{17}NO_8$ (303.27) Ber. C 47.53 H 5.65 N 4.62
Gef. C 47.48 H 5.60 N 4.64

**Beispiel 10**

**2-Aminomethyl-5-(α-D-glucopyranosyloxymethyl)-furan** (Formel **10**)

In einem 125 ml Rührautoklaven werden 5.76 g (0.02 mol) GMF in 50 ml mit Ammoniak bei 10°C gesättigtem Methanol (ca. 5.5 M) gelöst. Nach Zusatz von 1 g alkalischem Raney-Nickel leitet man Wasserstoff bis zu einem Druck von 100 atm ein und erwärmt den Ansatz auf 60°C . Die Temperatur wird 1 h konstant gehalten, danach läßt man über einen Zeitraum von 4 h Abkühlen und filtriert nach Entspannen des Reaktionsgefäßes vom Katalysator ab. Das Filtrat wird i. Vak. vollständig eingeengt, woraus ein aus Amin der Formel (**10**) bestehender Hartschaum resultiert.

$^1$H-NMR (300 MHz, [$D_6$]-DMSO + $D_2O$): δ = 3.10 (dd, 1 H, 4'-H), 3.19 (s, 2 H, 6-$H_2$), 3.24 (dd, 1 H, 2'-H), 3.45 (m, 2 H, 3'-H, 5'-H), 3.63 (m, 2 H, 6'-$H_2$). 4.38 (d, 1 H, 7-$H_a$), 4.52 (d, 1 H, 7-$H_b$), 4.77 (d, 1 H, 1'-H), 6.18 (d, 1 H, 4-H), 6.35 (d, 1 H, 3-H); $J_{3,4}$ = 2.9, $J_{7_a,7_b}$ = 12.8, $J_{1',2'}$ = 3.5, $J_{2',3'}$ = 9.6 Hz.

**Beispiel 11**

**5-Acetamidomethyl-2- (2',3',4',6'-tetra-*O*-acetyl-α-D-glucopyranosyloxymethyl)-furan** (Formel **11**)

Eine Lösung von 0.9 g (2 mmol) des Nitrils der Formel (**14**) in 50 ml Ethylacetat wird in Gegenwart von 100 mg 10proz. Pd auf Kohle bei Raumtemperatur hydriert. Nach 12 h ist vollständiger Umsatz erreicht (DC in Methanol/Chloroform, 2:1). Es wird vom Katalysator abfiltriert, mit heißem Methanol gewaschen (4 x 20 ml), und die methanolische Lösung tropfenweise mit Acetanhydrid (1.5 ml) versetzt, und über Nacht stehen gelassen. Abziehen des Methanols, Digerieren des Rückstandes mit Eiswasser, Ausschütteln mit Chloroform, Trocknen der vereinigten Extrakte über Magnesiumsulfat, und Entfernung des Lösungsmittels i. Vak. liefert 1.1 g (74 %) Produkt der Formel (**11**) als farblose Kristalle vom Schmp. 134 - 135°C; $[\alpha]_D^{20}$ = + 121° (c = 1.3, Chloroform).

$C_{22}H_{29}NO_{12}$ (499.48) Ber. 52.90 H 5.85 N 2.80
Gef. 52.93 H 5.60 N 2.77

Dasselbe Produkt wird auch durch Acetylierung des Amins der Formel (10) aus Beispiel 10 mit Pyridin/Acetanhydrid in 87proz. Ausbeute erhalten.

**Beispiel 12**

**5-(α-D-Glucopyranosyloxymethyl)-furan-2-carbonitril** (Formel **13**)

Eine Lösung von 1.0 g (3.5 mmol) GMF der Formel (**2**) in 30 ml DMSO wird auf 110°C erhitzt, Nach Zugabe von 310 mg (4.5 mmol) Hydroxylammoniumhydrochlorid wird die Temperatur über eine Zeitraum von 30 min (DC-Kontrolle mit 4:1 Acetonitril/Wasser) beibehalten und sodann rasch abgesenkt. Der Reaktionsansatz wird i. Vak. eingeengt, der Rückstand mit 30 ml Wasser verdünnt und mit Dichlormethan (2 x 20 ml) ausgeschüttelt. Die i. Vak. eingedampfte wäßrige Phase wird durch Elution von einer Kieselgelsäule (3 x 30 cm) mit Aceton gereinigt. Die das Nitril enthaltenden Fraktionen liefern beim Einengen 0.6 g (61 %) Nitril der Formel (**13**) als farblose Nadeln vom Schmp. 113°C und $[\alpha]_D^{20}$ = + 119° (c = 1, Methanol).

$^1$H-NMR (300 MHz, [$D_6$]-DMSO): δ = 3.04 (m, 1 H, 4'-H), 3.17 (m, 1 H, 2'-H), 3.27 - 3.58 (m, 4 H, 3'-H, 5'-H, 6'-$H_2$), 4.45 (t, 1 H, 6'-OH), 4.47 (d, 1 H, 7-$H_a$), 4.59 (d, 1 H, 7-$H_b$), 4.70 (d, 1 H, 1'-H), 4.77 (m, 2 H, OH), 4.87 (d, 1 H, OH), 6.54 (d, 1 H, 4-H), 7.51 (d, 1 H, 3-H); $J_{3,4}$ = 3.6, $J_{7_a,7_b}$ = 13.5, $J_{1',2'}$ = 3.6, $J_{6',6'-OH}$ = 5.7 Hz.

MS (FD): m/z =285 ($M^{\oplus}$), 286 ($M^{\oplus}$ +1).

$C_{12}H_{15}NO_7$ (285.25) Ber. C 50.53 H 5.30 N 4.91
Gef. C 50.49 H 5.32 N 4.86

**Beispiel 13**

**5-(2',3',4',6'-Tetra-*O*-acetyl-α-D-glucopyranosyloxymethyl)-furan-2-carbonitril** (Formel **14**)

Eine Suspension von 3.0 g (10 mmol) GMF-Oxim (Formel (**9**), Produkt von Beispiel 9) und 1.0 g frisch geschmolzenem Natriumacetat in 10 ml Acetanhydrid wird für 30 min bei Raumtemperatur gerührt. Die nunmehr klare Lösung wird sodann durch Zugabe von Salzsäure (4 ml 4 N HCl, 1.3 Molequiv. bezogen auf das eingesetzte Natriumacetat) schwach mineralsauer gemacht, und kurz (5 - 8 min) auf 60°C erwärmt. Einrühren in Eiswasser, Extraktion mit Dichlormethan und Eindampfen der vereinigten Extrakte liefert 3.5 g (78 %) des Nitrils der Formel (**14**) als chromatographisch einheitlichen Sirup mit $R_f$ = 0.31 (DC in Toluol/Aceton, 4:1) und $[\alpha]_D^{20}$ = + 135° (c = 0.7, Chloroform).

$^1$H-NMR (300 MHz, $CDCl_3$): δ = 2.01, 2.04, 2.06, 2.10 (4 s, je 3 H, Ac-$CH_3$), 4.02 - 4.10 (m,2 H, 5'-H, 6'-$H_a$), 4.27 (dd. 1 H, 6'-$H_b$), 4.59 (d, 1 H, 7-$H_a$), 4.69 (d, 1 H, 7-$H_b$), 4.86 (dd, 1 H, 2'-H), 5.08 (dd, 1 H, 4'-H), 5.18 (d, 1 H, 1'-H), 5.47 (dd, 1 H, 3'-H), 6.51 (d, 1 H, 4-H); $J_{3,4}$ = 3.5, $J_{7_a,7_b}$ = 13.4, $J_{1',2'}$ = 3.8, $J_{2',3'}$ = 10.3, $J_{3',4'}$ = 9.7, $J_{4',5'}$ = 10.0, $J_{5',6'_b}$ = 4.5, $J_{6'_a,6'_b}$ = 12.5 Hz.

$^{13}$C-NMR (75.5 MHz, $CDCl_3$): δ = 20.5, 20.6, 20.7 (4 C, Ac-$CH_3$), 61.5, 61.8 (2 C, C-7, C-6'), 67.9, 68.5, 69.8, 70.7 (4 C, C-2', C-3', C-4', C-5'), 95.3 (C-1'), 111.1 (C-5), 111.2 (C-4), 122.7 (C-3), 125.6 (C-2), 155.7 (C-6), 169.5, 169.9, 170.1, 170.5 (4 C, Ac-CO).

MS (FD): m/z = 453 ($M^{\oplus}$), 454 ($M^{\oplus}$ +1).

$C_{20}H_{23}NO_{11}$ (453.41) Ber. C 52.98 H 5.11 N 3.09
Gef. C 52.99 H 4.87 N 3.06

**Beispiel 14**

**2-(Benzoylvinyl)-5-(α-D-glucopyranosyloxymethyl)-furan** (Formel **15**)

Eine Lösung von 4.5 g GMF (15 mmol) und 1.8 g (15 mmol) Acetophenon in 50 ml Ethanol wird auf 0°C abgekühlt und unter Rühren tropfenweise mit 15 ml 10proz. NaOH versetzt. Nach 30 min wird mit 50 ml Wasser verdünnt, mit Dichlormethan ausgeschüttelt (3 x 60 ml), die vereinigten organischen Extrakte mit gesättigter Kaliumhydrogensulfat-Lösung und wenig Wasser neutral gewaschen und getrocknet ($MgSO_4$). Beim Einengen der Lösung tritt Kristallisation ein: 4.1 g (70 %) hellgelbe Kristalle vom Schmp. 127°C ; $[\alpha]_D^{20}$ = +111° (c = 0.9, Methanol).

$^1$H-NMR (300 MHz, $CDCl_3$): δ = 3.48 - 3.78 (m, 7 H, 2'-H, 3'-H, 4'-H, 5'-H, 6'-$H_2$, OH), 4.10 (d, 1 H, OH), 4.50 (d, 1 H, 9-$H_a$), 4.57 (d, 1 H, 9-$H_b$), 4.75 (s, 1-H, OH), 4.92 (d, 1 H, 1'-H), 4.98 (s, 1 H, OH), 6.39 (d, 1 H, 4-H), 6.56 (d, 1 H, 3-H), 7.28 - 7.52 (m, 5 H, $COC_6H_5$), 7.93 (d' 2 H, 6-H, 7-H); $J_{3,4}$ = 3.3, $J_{9_a,9_b}$ = 13.5, $J_{1',2'}$ = 3.2 Hz.

$^{13}$C-NMR (75.5 MHz, DMSO-$d_6$): δ = 60.2 (C-9), 60.8 (C-6'), 70.1 (C-4'), 71.4 (C-2'), 73.1 (C-3',C-5'), 98.0 (C-1'), 112.4 (C-4), 118.0 (C-3), 118.5, 128.2, 130.4, 133.0 und 137.4 ($C_6H_5$), 128.8 (C-6, C-7), 151.0 (C-5), 154.8 (C-2), 188.5 (C=O).

MS (FD): m/z = 390 ($M^{\oplus}$).

$C_{20}H_{22}O_8$ (390.38) Ber. C 61.53 H 5.68
Gef. C 61.49 H 5.65

**Beispiel 15**

**2-(Dicyanovinyl)-5-(α-D-glucopyranosyloxymethyl)-furan** (Formel **16**)

1 g (3.5 mmol) GMF der Formel (1) und 230 mg (3.5 mmol) Malonsäuredinitril werden in 10 ml Methanol gelöst und mit 600 mg Aluminiumoxid (24 h bei 120°C getrocknet) versetzt. Nachdem 3 h bei Raumtemperatur gerührt wurde (DC-Kontrolle mit Acetonitril/Wasser, (4:1) wird vom Katalysator abfiltriert und das Lösungsmittel i. Vak. abgezogen. Nach Reinigen des Rohprodukts durch Elution von einer Kieselgelsäule (3 x 20 cm) mit Aceton kristallisieren 780 mg (66 %) Produkt der Formel (**16**) beim Einengen des Lösungsmittels in Form hellgelber Nadeln vom Schmp. 138°C und $[\alpha]_D^{20}$ = +104° (c = 0.6, Methanol).

$^1$H-NMR (300 MHz, $D_2O$): δ = 3.44 (dd, 1 H, 4'-H), 3.58 (dd, 1 H, 2'-H), 3.68-3.76 (m, 4 H, 3'-H, 5'-H, 6'-$H_2$), 4.77 (d, 1 H, $7_a$-H), 4.84 (d, 1 H, $7_b$-H), 5.09 (d, 1 H, 1'-H), 6.83 (d, 1 H, 4-H), 7.39 (d, 1 H, 3-H), 7.91 (s, 1 H, 6-H); $J_{3,4}$ = 3,7 , $J_{7_a,7_b}$ = 13.5, $J_{1',2'}$ = 3.7, $J=_{2',3'}$ = 9.8 Hz.

IR (KBr): ν= 2240 $cm^{-1}$ (C≡N).

MS (FD, 10 mA): m/z = 336 ($M^{\oplus}$).

$C_{15}H_{17}NO_9$ (336.30) Ber. C 53.57 H 4.80 N 8.33
Gef. C 53.42 H 4.83 N 8.25

**Patentansprüche**

1. Verfahren zur Herstellung von 5-(α-D-Glucopyranosyloxymethyl)-furan-2-carboxaldehyd,
**dadurch gekennzeichnet,**
daß man Isomaltulose, wasserfrei oder mit einem Mol Kristallwasser, in einem stark polaren aprotischen Lösungsmittel löst (10 bis 100g Isomaltulose / 100g Lösungsmittel) und die Lösung
   - entweder
     in Gegenwart eines sauren Ionenaustauschers in der $H^+$ -Form als Katalysator während 0.1 bis 24 Stunden auf 70 bis 150°C batchweise erhitzt, wobei das Verhältnis von Isomaltulose: Ionenaustauscher 20:1 bis 10:1 beträgt.
   - oder
     über eine mit dem gleichen Ionenaustauscher gefüllte Säule bei Temperaturen zwischen 70 und 150°C und Verweilzeiten zwischen 20 sec. und 60 min perkoliert.

2. Derivate von 5-(α-D-Glucopyranosyloxymethyl)-furan-2-carboxaldehyd und Folgeprodukte gemäß der allgemeinen Formel (I)

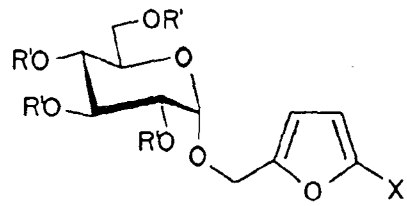

(I)

in der
R' ein Wasserstoffatom, einen Acyl-Rest oder einen Alkyl-Rest darstellt und X-$CH_2OH$, -COOH, -C(H)=NOH, -CN, -$CH_2NHR$", -CH=CR"R" ' (wobei R" ein Wasserstoffatom, ein Acyl-Rest oder Alkyl-Rest mit bis zu 20 C-Atomen ist, und R"' ein Wasserstoffatom, -$NO_2$, -CN, -COOAlkyl oder Acyl) bedeutet, insbesondere die Verbindungen
2- (α-D-Glucopyranosyloxymethyl)-5-hydroxymethylfuran,
5- (α-D-Glucopyranosyloxymethyl)-furan-2-carboxaldehydoxim (GMF-Oxim),
5-(α-D-Glucopyranosyloxymethyl)-furan-2-carbonsäure,
5-(2',3',4',6',-Tetra-O-acetyl-α-D-glucopyranosyloxymethyl)-furan-2-carbonitril,
5-Acetamidomethyl-2-(α-D-glucopyranosyloxymethyl) -furan,
2-(Benzoylvinyl)-5-(α-D-glucopyranosyloxymethyl)-furan und
2-(Dicyanovinyl)-5-(α-D-glucopyranosyloxymethyl)-furan.

3. Verfahren zur Herstellung der O-Acylderivate des GMF und seiner Abkömmlinge,
**dadurch gekennzeichnet,**
daß man die jeweiligen nicht O-acylierten Ausgangsverbindungen mit Acylierungsmitteln in wasserfreien organischen Lösungsmitteln behandelt.

4. Weiterverarbeitung von GMF und seiner Derivate zur Gewinnung der zugehörigen Alkohole, Carbonsäuren und Oxime,
**dadurch gekennzeichnet,**
daß man die Ausgangsprodukte in an sich bekannter Weise reduziert, oxidiert oder oximiert.

5. Verwendung der Verbindungen gemäß Anspruch 2 zur Herstellung von oberflächenaktiven Stoffen.

## Claims

1. Method for producing 5-(α-D-glucopyranosyloxymethyl)-furan-2-carboxaldehyde, characterized in that isomaltulose, anhydrous or with one mole of water of crystallization, is dissolved in a greatly polar aprotic solvent (10 to 100g isomaltulose / 100g solvent) and the solution is
   - either
     heated in the presence of an acidic ion exchanger in the $H^+$-form as catalyst for 0.1 to 24 hours to 70 to 150°C in batches, with the ratio of isomaltulose to ion exchanger amounting to 20:1 to 10:1,
   - or
     percolated over a column filled with the same ion exchanger at temperatures between 70 and 150°C and dwell times between 20 sec. and 60 min.

2. Derivatives of 5-(α-D-g.ucopyranosyloxymethyl)-furan-2-carboxaldehyde and secondary products according to the general formula (I)

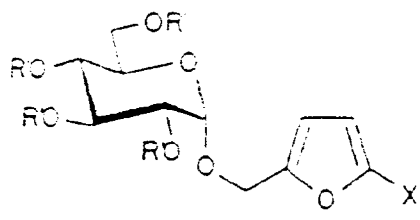

(I)

in which
R' represents a hydrogen atom, an acyl residue or an alkyl residue and signifies X-$CH_2OH$, -COOH, -C(H)=NOH, -CN, -$CH_2NHR$'', -CH=CR''R''' (with R'' being a hydrogen atom, an acyl residue or alkyl residue with up to 20 C-atoms, and
R''' a hydrogen atom, -$NO_2$, -CN, -COOalkyl or acyl), in particular the compounds
2-(α-D-glucopyranosyloxymethyl)-5-hydroxymethylfuran,
5-(α-D-glucopyranosyloxymethyl)-furan-2-carboxaldehydoxime (GMF-oxime),
5-(α-D-glucopyranosyloxymethyl)-furan-2-carboxylic acid,
5-(2',3',4',6',-tetra-O-acetyl-α-D-glucopyranosyloxymethyl)-furan-2-carbonitrile,
5-acetamidomethyl-2-(α-D-glucopyranosyloxymethyl)-furan,
2-(benzoylvinyl)-5-(α-D-glucopyranosyloxymethyl)-furan and
2-(dicyanovinyl)-5-(α-D-glucopyranosyloxymethyl)-furan.

3. Method for producing the O-acyl derivatives of the GMF and its derivatives, characterized in that the respective starting compounds which are not O-acylated are treated with acylation means in anhydrous organic solvents.

4. Further processing of GMF and its derivatives for obtaining the associated alcohols, carboxylic acids and oximes, characterized in that the starting products are reduced, oxidized or oximized in a manner known in itself.

5. Use of the compounds according to claim 2 for producing surface-active substances.

## Revendications

1. Procédé de préparation de 5-(α-D-Glucopyranosyloxyméthyl)-furane-2-carboxaldéhyde,
**caractérisé** en ce qu'on dissout de l'isomaltulose, anhydre ou avec une mole d'eau de cristallisation, dans un solvant aprotique fortement polaire (10 à 100 g d'isomaltulose par 100 g de solvant), et en ce qu'on soumet la solution soit :
   - en présence d'un échangeur d'ions acide sous la forme $H^+$ en tant que catalyseur pendant 0,1 à 24 heures à un chauffage de 70 à 150°C en charges successives, la proportion d'isomaltulose/échangeur d'ions étant de 20/1 à 10/1,
   - ou
   à une percolation dans une colonne remplie du même échangeur d'ions à des températures entre 70 et 150°C et avec des temps de séjour entre 20 secondes et 60 minutes.

2. Dérivés de 5-(α-D-glucopyranosyloxyméthyl)-furan-2-carboxaldéhyde et produits de filiation selon la formule générale (I)

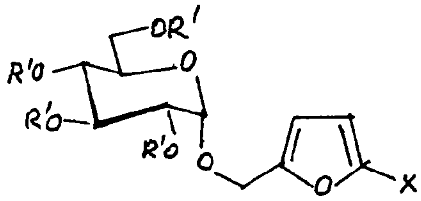

(I)

dans laquelle :
R' représente un atome d'hydrogène, un reste acyl- ou un reste alkyl- et X-$CH_2OH$, -COOH, -C(H)=NOH, -CN, -$CH_2NHR$", -CH=CR"R" ' (où R" est un atome d'hydrogène, un reste acyl- ou un reste alkyl- avec jusqu'à 20 atomes carbone, et R'" signifie un atome d'hydrogène, -$NO_2$; -CN, -COOalkyle ou acyle), en particulier les composés :
2-(α-D-Glucopyranosyloxyméthyl)-5-hydroxyméthylfurane,
5-(-α-Glucopyranosyloxyméthyl)-furane-2-carboxaldéhydoxime (oxime GMF),
5-(α-D-glucopyranosyloxyméthyl)-furane-2-acide carbonique,
5-(2', 3', 4', 6'), -tetra-O-acétyl-α-D-glucopyranosyloxyméthyl)-furane-2-carbonitrile,
5-acétamidométhyl-2-(α-D-glucopyranosyloxyméthyl)-furane
2-(benzoylvinyl)-5-(α-D-glucopyranosyloxyméthyl)-furane et
2-(dicyanovinyle)-5-(α-D-glucopyranosyloxyméthyl)-furane

3. Procédé de préparation des dérivés O-acyl du GMF et de ses produits de filiation,
**caractérisé** en ce qu'on traite les composés de départ non O-acylisés avec des agents d'acylisation dans des solvants organiques anhydres.

4. Transformation du GMF et de ses dérivés pour l'obtention des alcools, acides carboniques et oximes correspondants,
**caractérisée** en ce qu'on soumet les produits de départ à une réduction, une oxydation ou une oximisation d'une manière connue en soi.

5. Utilisation des composés selon la revendication 2 à la préparation de substances tensio-actives.

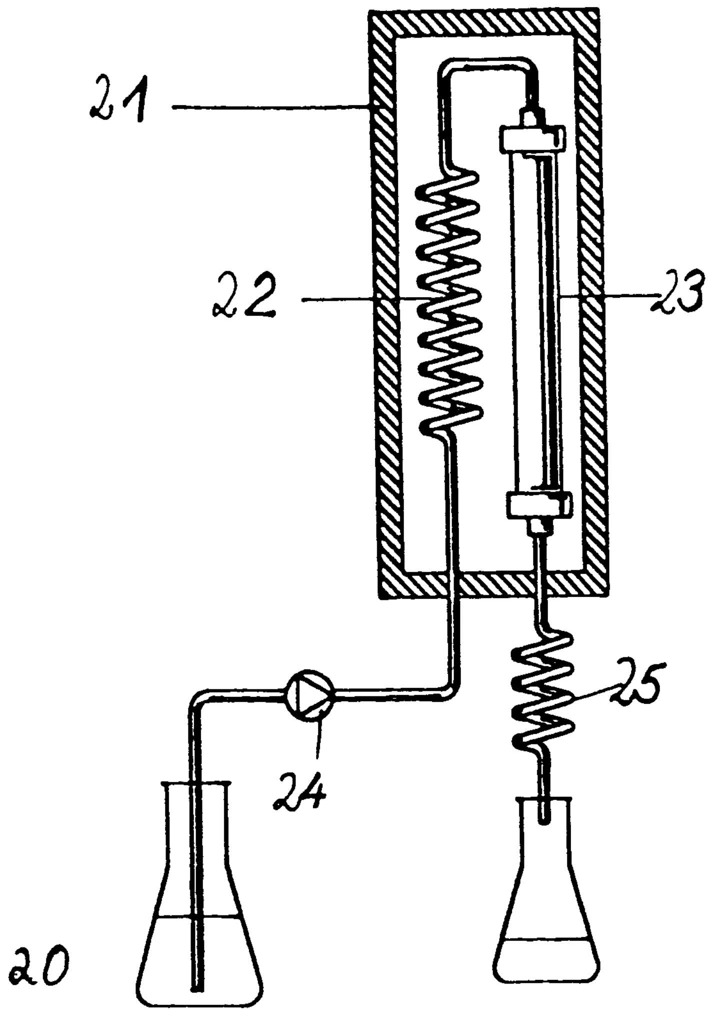


Abbildung 1